# EUROPÄISCHE PATENTSCHRIFT

(11) **EP 0 734 725 B1**
(45) Veröffentlichungstag und Bekanntmachung des Hinweises auf die Patenterteilung: **09.07.2003**
(21) Anmeldenummer: 96103131.7
(22) Anmeldetag: 01.03.1996
(51) Int. Cl.: A61K 33/26, A61P 31/00

(54) **Adjuvans auf der Basis kolloidaler Eisenverbindungen**
Adjuvants containing colloidal iron compounds
Adjuvants contenant des composés de fer colloidaux

(30) Priorität: 27.03.1995 DE 19511276
(43) Veröffentlichungstag der Anmeldung: 02.10.1996
(73) Patentinhaber: Bio-Products & Bio-Engineering Aktiengesellschaft, 1010 Wien (AT)
(72) Erfinder: Eibl, Johann, Dr., 1180 Wien (AT); Leibl, Heinz, Dr., 1120 Wien (AT); Mannhalter, Josef, Prof. Dr., 1050 Wien (AT)
(74) Vertreter: Schwarz, Albin, Dr.

(56) Entgegenhaltungen:
- EP-A- 0 363 835
- EP-A- 0 465 081
- WO-A-91/09622
- WO-A-94/15635
- DE-B- 1 041 647
- US-A- 1 249 558
- US-A- 4 452 773
- GUPTA R.K. ET AL: "Adjuvants - a balance between toxicity and adjuvanticity" VACCINE, Bd. 11, Nr. 3, 1993, Seiten 293-306, XP002111596
- WARREN J. ET AL: "The adjuvant effect of powdered ferric oxide: enhancement of response to Mycoplasma Pneumoniae and respiratory syncytial virus vaccines" THE JOURNAL OF IMMUNOLOGY, Bd. 102, Nr. 5, 1969, Seiten 1300-1308, XP002111597
- J. FALBE AND M.REGITZ: "RÖMPP CHEMIE LEXIKON, Seiten 2299-2304, ", 1999, GEORG THIEME VERLAG SUTTGART, NEW YORK
- PATENT ABSTRACTS OF JAPAN, Band 11, Nr. 60 (C-405), 24. Februar 1987 & JP 61 218522 A

## Beschreibung

Die vorliegende Erfindung betrifft die Verwendung kolloidaler Eisenverbindungen als Adjuvantien sowie diese enthaltende pharmazeutische Zusammensetzungen.

Unter einem Adjuvans versteht man ein Hilfsmittel, das die Immunogenität eines Antigens, mit dem es gemeinsam verabreicht wird, erhöht oder die Qualität der Immunantwort beeinflusst. Adjuvantien werden mit der Absicht verabreicht, die Immunogenität eines Antigens zu verbessern, was zu einer erhöhten Antikörperbildung bzw. zu einer stärkeren zellvermittelten Immunantwort in bezug auf das Antigen führt.

Gebräuchliche Adjuvantien sind Aluminiumverbindungen, lipidhaltige Verbindungen sowie inaktivierte Mycobakterien. Zu den effizientesten Adjuvantien zählt das sogenannte Freundsche Adjuvans, eine Wasser-in-Öl-Emulsion. Besonders wirksam ist dieses Adjuvans, wenn Mycobakterien in der Emulsion zusammen mit dem Antigen suspendiert sind (*Freundsches komplettes Adjuvans*). Ein Nachteil dieses Adjuvans bei der Anwendung am Menschen ist jedoch das mögliche Auftreten einer chronischen Entzündung. Emulsionen ohne Mycobakterien *(Freundsches inkomplettes Adjuvans*) führen hingegen zu geringeren Nebenwirkungen und werden deshalb bereits am Menschen angewandt.

Eine weitere Gruppe von Adjuvantien stellen Suspensionen von Mineralsalzen dar, an die das entsprechende Antigen adsorbiert ist. Die gebräuchlichsten Adjuvantien dieser Art sind Verbindungen von Aluminium, wie Aluminiumhydroxid, Aluminiumphosphat oder Aluminiumsulfat.

Auch bei gewissen Lymphokinen konnte Adjuvansaktivität nachgewiesen werden. So ist beispielsweise von Good et al., J.Immunol.141, 972-977, 1988, die Erhöhung der Immunantwort gegen ein Malaria Antigen durch rekombinantes humanes Interleukin-2, welches an Aluminium adsorbiert ist, gezeigt worden. Nakamura et al., Nature 307, 381-382, 1984, zeigten eine 2- bis 5-fache Erhöhung der Antikörperproduktion gegen unterschiedliche Antigene durch gamma-Interferon.

Neben der Art der immunologischen Wirkungsweise stellt die Pharmakokinetik bzw. Bioabbaubarkeit des jeweiligen Stoffes ein weiteres Kriterium für dessen Eignung als Adjuvans dar. So etwa sind Stoffe, die im Organismus einem Stoffwechsel unterliegen, im allgemeinen weniger toxisch und lokal recht gut verträglich. Dies gilt insbesondere für Eisen, welches als wichtigstes Spurenelement in menschlichen und tierischen Organismen vorkommt. Dieses Eisen kommt üblicherweise in Form von Eisensalzen vor, welche nach Aufnahme im Körper in Ionenform vorliegen und sehr gut resorbiert werden können.

Neben den vorstehend erwähnten Adjuvantien sind auch weitere Stoffe mit Adjuvans-Eigenschaften bekannt. In der EP-B-0363 835 wird ein Zinkhydroxidgel oder Eisenhydroxidgel zur Adjuvierung einer Antigenlösung beschrieben. Die Antigenlösung enthält z.B. 1-45 % (v/v) Eisenhydroxidgel und gegebenenfalls Lecithin. Das Adjuvans wird ausgehend von FeCl₃-Lösungen nach Sterilfiltration durch Zugabe von NaOH oder KOH bis zu einem pH-Wert von 6,0 bis 7,8 als Präzipitat erhalten. Durch eine Ultraturrax-Behandlung wird das ausgefallene Eisenhydroxidgel gegebenenfalls homogenisiert. Durch diese Behandlung entsteht aber kein kolloidales Eisen-III-hydroxid, sondern dieses liegt, wie im Vergleichsversuch 1 gezeigt wird, immer noch als Gel vor. Das so hergestellte Eisenhydroxidgel induziert vorwiegend eine humorale Immunantwort. Ausserdem wird im Vergleichsversuch 2 gezeigt, dass sich das gemäss EP-363 835 erhaltene Gel in seiner Proteinadsorption anders verhält als die erfindungsgemässe kolloidale Lösung, welche eine deutlich grössere Menge an Protein zu binden vermag.

US-4,452,773 beschreibt ferromagnetische Partikel von Fe₃O₄, die mit wasserlöslichen Polysacchariden beschichtet und von kolloidaler Grösse sind. Die Partikel weisen einen Durchmesser zwischen 100 und 700 Å auf, besitzen ein magnetisches Moment, sind mono-dispergierbar und unter physiologischen Bedingungen stabil gegenüber Aggregation und Degradation. Sie sind aufgrund ihrer ferromagnetischen Eigenschaften für die Markierung von Zellen oder anderen biologischen Materialien geeignet. Somit dienen sie aufgrund ihrer unterschiedlichen Ausgestaltung und Eigenschaften einem völlig anderen Zweck, wie insbesondere zur Markierung von Biomolekülen oder ganzen Zellen.

In der WO-A-94 15 635 wird eine pharmazeutische Zusammensetzung beschrieben, die einen partikulären Proteinkomplex aus einer an ein nicht-replizierendes Protein-Antigen gebundenen partikulären Komponente enthält. Die partikuläre Komponente kann u.a. ein Metall sein, bevorzugt ein Metall aus den Übergangselementen Scandium bis Kupfer, oder ein entsprechendes Metalloxid, insbesonders Eisenoxid. Das Eisen(III)oxid liegt als magnetisches γ-Oxid vor.

Die im Stand der Technik beschriebenen Adjuvantien auf Eisenbasis weisen den Nachteil auf, dass einerseits die Oberflächen der Eisenverbindungen insgesamt relativ klein sind, was eine geringe Adsorption des Antigens impliziert, und dass andererseits diese Verbindungen vom Organismus schlecht bzw. verzögert resorbiert werden.

Aufgabe der vorliegenden Erfindung ist es, ein Adjuvans zur Verfügung zu stellen, welches gut verträglich ist, und mit dem die Immunantwort verstärkt werden kann.

Diese Aufgabe wird erfindungsgemäss mit einer Eisenverbindung in kolloidaler Form gemäß den Ansprüchen gelöst.

Überraschenderweise wurde nämlich gefunden, dass sich die anmeldungs gemäßen Eisenverbindungen in kolloidaler Form sehr gut für die Verwendung zur Adjuvierung einer Antigenlösung eignen; die erfindungsgemäss erhaltenen Adjuvantien sind überaus stabil, weisen gute adsorbierende und Protein-stabilisierende Eigenschaften auf und verstärken die Immunantwort, wodurch ein Schutz gegenüber Infektionen gewährleistet wird.

Das erfindungsgemässe Adjuvans enthält eine Eisenverbindung in kolloidaler Form. Das Eisen liegt in der Eisenverbindung bevorzugt in dreiwertiger Form vor, ist vorzugsweise eine anorganische Eisenverbindung und ist vorzugsweise im wesentlichen Eisenhydroxid. Die erfindungsgemässe Eisenverbindung kann auch ein Gemisch von Eisenhydroxiden und Eisenoxiden darstellen, die in verschiedenen Hydratisierungsstufen und Modifikationen vorliegen können. In dem Kolloid können auch entsprechende Anionen der ursprünglich verwendeten Eisenverbindung, beispielsweise Citrationen, enthalten sein.

Unter kolloidaler Form versteht man Stoffe in einem Verteilungszustand, bei dem das Vorhandensein von dispersen Teilchen makroskopisch nicht erkennbar ist. Eine kolloidale Lösung, auch als Sol bezeichnet, kann normale Filter und auch Sterilfilter passieren, während sie von Ultrafiltern zurückgehalten wird. Aufgrund der Art der Beweglichkeit der Teilchen und der Art des Zusammenhalts der dispergierten Substanz zählen die erfindungsgemässen kolloidalen Lösungen zu den inkohärenten Systemen; in solchen Systemen ist der Grossteil der Teilchen als "kinetische Einheit" frei beweglich und hängt mit keinem anderen Teilchen zusammen.

Magnetische Eigenschaften der erfindungsgemässen Eisenverbindung sind nicht notwendig, da die Verbindung nicht zur Markierung von Biomolekülen, sondern überraschenderweise als Adjuvans verwendet werden kann.

Im Gegensatz zu einer Verbindung, die zur Markierung verwendet wird, muss die Eisenverbindung gemäss der vorliegenden Erfindung nicht notwendigerweise an das immunogene Agens gebunden vorliegen, sondern kann auch getrennt von diesem, z.B. gleichzeitig oder aufeinander folgend, verabreicht werden.

In einer bevorzugten Ausführungsform besitzt das erfindungsgemässe Adjuvans keine magnetischen, insbesondere keine ferromagnetischen Eigenschaften bzw. besitzt das erfindungsgemässe Adjuvans diese Eigenschaften nur in einem geringen Ausmass.

Die erfindungsgemässe Lösung ist bevorzugt bei 4°C haltbar und stabil. Unter stabiler kolloidaler Lösung wird eine Lösung verstanden, deren Teilchen bei Stehenlassen auch nach mehreren Stunden oder Tagen nicht sedimentieren.

Kolloidale Lösungen sind weiters durch die Teilchengrösse gekennzeichnet, welche etwa zwischen 1 nm und 500 nm liegt. Die Teilchengrösse der erfindungsgemässen Lösung liegt zwischen 100 nm und 500 nm, besonders bevorzugt um ca. 200 nm. Die Partikelgrössenbestimmung der kolloidalen Lösung kann nach gängigen Methoden erfolgen, beispielsweise nach dem Prinzip der dynamischen Lichtstreuung.

Das erfindungsgemässe Adjuvans besteht aus kolloidalen Teilchen definierter Zusammensetzung, welche besonders gute adsorbierende Eigenschaften bzw. eine hohe Bindungskapazität für Antigene, z.B. Proteine, besitzen. Aufgrund der grossen Gesamtoberfläche des erfindungsgemässen Adjuvans kann bereits mit einer geringeren Menge an Eisenverbindung mehr Protein adsorbiert werden, als dies mit üblichen Trägersystemen der Fall ist. Dadurch lassen sich u.a. auch mögliche Nebenwirkungen verringern.

Die Viskosität der erfindungsgemässen kolloidalen Lösung ist mit der einer Proteinlösung bzw. 10 - 20%igen Glycerinlösung vergleichbar und beträgt etwa 1 bis 5 cP.

Das erfindungsgemässe Adjuvans liegt als wässerige, kolloidale Lösung oder auch als Wasser-in-Öl-Emulsion vor. Die kolloidale Lösung weist vorzugsweise einen pH-Wert zwischen 4,0 bis 11,0 auf, bevorzugt zwischen 6,8 bis 9,0 auf, mehr bevorzugt 7,0 bis 8,5, am meisten bevorzugt 8,0.

Die Wahl des pH-Wertes kann u.a. von dem verwendeten Protein bzw. Antigen abhängig sein.

Die Wasser-in-Öl-Emulsion besteht beispielsweise aus einer Eisenverbindung in kolloidaler Form, Freundschem inkomplettem Adjuvans und Wasser. Das Verhältnis von wässerigem Kolloid zu Öl beträgt beispielsweise 1:1, es sind aber auch alle anderen Mischungsverhältnisse möglich.

Das erfindungsgemässe Adjuvans oder eine dieses enthaltende pharmazeutische Zusammensetzung liegt vorzugsweise in einer für die parenterale Verabreichung geeigneten Form vor. Die parenterale Verabreichung kann beispielsweise intramuskulär, subkutan, intradermal oder intraperitoneal erfolgen. Das Adjuvans ist auch geeignet für die Stimulation der Mukosaimmunität, wie sie durch mukosale, beispielsweise durch orale, intranasale, intravaginale oder intrarektale Verabreichung induziert wird. Das Adjuvans kann für diese Zwecke direkt, ohne weiter Vorbehandlung, aber auch in Kapseln oder Liposomen eingeschlossen, verwendet werden. Das erfindungsgemässe Adjuvans bzw. die fertige pharmazeutische Zusammensetzung kann auch durch Lyophilisation haltbar gemacht werden. Nach Rekonstitution des Lyophilisats wird eine Lösung mit den erfindungsgemässen Eigenschaften erhalten.

Das erfindungsgemässe Adjuvans kann zusätzlich wenigstens ein Interleukin, Interferon oder anderes Cytokin enthalten, beispielsweise Interleukin-2,4,5,7,12 oder Interferon-γ. Auch Mischungen davon sind möglich. Das entsprechende Cytokin bzw. die Cytokinmischung ist vorzugsweise an das Adjuvans adsorbiert. Adjuvans und Cytokin sind durch kovalente oder nicht kovalente Assoziation verbunden.

Das erfindungsgemässe Adjuvans ist dadurch erhältlich, dass man eine Lösung eines Eisensalzes in einem Lösungsmittel durch Einstellen eines geeigneten pH-Wertes in eine Eisenverbindung in kolloidaler Form überführt. Als Eisensalz wird vorzugsweise ein Salz einer schwachen Säure, und insbesonders Eisencitrat verwendet.

Das Lösungsmittel ist vorzugsweise Wasser oder ein Gemisch aus Wasser und einem vorzugsweise mit Wasser mischbaren organischen Lösungsmittel, wie beispielsweise Ethanol oder Glycerin.

Unter einer schwachen Säure wird eine Säure verstanden, die in Lösung unvollständig dissoziiert vorliegt. Der entsprechende pKₐ-Wert ist grösser als 2,0 und liegt bevorzugt zwischen 20,0 und 2,0. Entsprechende Salze sind beispielsweise Pyruvate, Acetate oder Tartrate, und insbesondere Citrate.

Das Adjuvans kann durch Einstellen eines geeigneten pH-Wertes erhalten werden, wobei der pH-Wert bevorzugt zwischen 7,0 und 9,0, und insbesonders bei ca. 8,0 liegt. Das Einstellen des pH-Wertes kann mit geeigneten alkalischen Lösungen, z.B. mit NaOH, KOH, Ammoniak oder Bicarbonat erfolgen.

Ein erfindungsgemässes Adjuvans in Form einer Wasser-in-Öl-Emulsion kann z.B. durch Emulgieren einer erfindungsgemässen wässrigen kolloidalen Lösung der Eisenverbindung mit einer geeigneten Ölkomponente, z.B. der ölkomponente von Freund's Adjuvans, oder mit Freund's Adjuvans, auf an sich bekannte Weise hergestellt werden.

Zur Herstellung eines erfindungsgemässen Adjuvans, das zusätzlich wenigstens ein Interleukin, Interferon, ein anderes Cytokin oder Mischungen davon enthält, kann das wie vorstehend beschriebene Adjuvans, vorzugsweise als wässerige kolloidale Lösung, mit einer Proteinlösung, vorzugsweise einer wässerigen Proteinlösung, vermischt werden.

Das erfindungsgemässe Adjuvans wird vorzugsweise sterilfiltriert und/oder in ein Lyophilisat übergeführt.

Gegenstand der Erfindung ist auch eine pharmazeutische Zusammensetzung gemäss den Ansprüchen, die dadurch gekennzeichnet ist, dass sie wenigstens ein Protein, Peptid, Polysaccharid und/oder eine Nukleinsäure abgeleitet von einem Virus, Bakterium undoder Parasiten, und ein erfindungsgemässes Adjuvans, gegebenenfalls zusammen mit einem geeigneten pharmazeutischen Träger und/oder Verdünner, enthält.

Das Virus ist bevorzugt ausgewählt aus der Gruppe bestehend aus Retroviren, Hepatitisviren, Flaviviren, Herpesviren, Influenza Viren, Poxviren, Morbilliviren, Enteroviren (Poliovirus) oder Paramyxoviren. Das Virus liegt bevorzugt als Ganzvirus, virus-ähnliches Partikel oder Virus-Spaltprodukt vor. Virus-ähnliche Partikel unterscheiden sich von einem Ganzvirus durch das Fehlen genetischer Information, enthalten also keine DNA. Das Virus-ähnliche Partikel kann aus Proteinen der äusseren und, wenn vorhanden, inneren Virushülle sowie weiteren Virusproteinen bestehen.

Virus-Spaltprodukte können Proteine oder Derivate davon, Polysaccharide, Peptide oder Nukleinsäuren abgeleitet von einem Virus sein und entweder aus nativem Virus stammen oder mittels rekombinanter Techniken hergestellt werden. Das Ganzvirus ist vorzugsweise inaktiviert, attenuiert oder ein rekombinantes Virus. Die Inaktivierung kann nach gängigen Methoden erfolgen, beispielsweise durch chemische Behandlung.

Die Attenuierung kann beispielsweise durch Punktmutation, ortsspezifische Mutagenese oder Deletion erfolgen. Nach üblichen Methoden können die Viren oder die Virusspaltprodukte auch rekombinant hergestellt werden. Als Vektoren werden hierfür Viren, wie beispielsweise, aber nicht ausschliesslich, Vacciniaviren oder Adenoviren, Plasmide, Cosmide oder Phagen, verwendet; das Vehikel ist mit entsprechenden Promotoren, Markern usw. ausgestattet.

Das Bakterium ist bevorzugt ausgewählt aus der Gruppe bestehend aus E.coli, Bordetella, Borrelia, Pseudomonas, Haemophilus, Mycobakterium, Streptokokken, Salmonellen, Helicobacter oder Clostridium. Das Bakterium kann als natives, rekombiniertes oder inaktiviertes Bakterium vorliegen oder aus unterschiedlichen Spaltprodukten bestehen. Bakterielle Spaltprodukte können Proteine, Peptide, Polysaccharide oder Nukleinsäuren von Bakterien darstellen.

Der Parasit ist bevorzugt ausgewählt aus der Gruppe bestehend aus Amaebida, Trypanosoma oder Plasmodium.

Die erfindungsgemässe pharmazeutische Zusammensetzung ist vorzugsweise eine Impfstoffzusammensetzung und enthält bevorzugt wenigstens ein Virus, Bakterium und/oder einen Parasiten bzw. ein Antigen eines Virus, Bakteriums oder Parasiten. Es sind also alle entsprechenden Mischungen daovn ebenfalls möglich. Das entsprechende Antigen aus Viren, Bakterien oder Parasiten wird aus denselben Gruppen ausgewählt, wie sie vorstehend für die pharmazeutische Zusammensetzung beschrieben sind.

Die pharmazeutische Zusammensetzung ist bevorzugt für die parenterale oder mukosale Verabreichung geeignet und kann zusätzlich ein Interleukin, Interferon, ein anderes Cytokin oder Mischungen davon enthalten.

Als pharmazeutische Träger und Verdünner können z.B. für Adjuvans-Zubereitungen übliche Träger und Verdünner verwendet werden. Der Adjuvans-Gehalt der pharmazeutischen Zusammensetzungen und die Menge ihrer Verabreichung entsprechen in der Regel ebenfalls den hierfür üblichen Bedingungen.

Aufgrund seiner Eigenschaften ist das erfindungsgemässe Adjuvans bzw. die daraus hergestellte pharmazeutische Zusammensetzung sehr gut zur prophylaktischen oder therapeutischen Behandlung von Erkrankungen, die durch verschiedene Pathogene hervorgerufen werden, geeignet.

Solche Erkrankungen können beispielsweise sein: Frühsommer-Meningoenzephalitis, Lyme Krankheit, AIDS, Herpes-Infektionen, Infektionen mit z.B. Pseudomonas oder Borrelien und weitere.

Mit den erfindungsgemässen Adjuvantien bzw. den daraus hergestellten pharmazeutischen Zusammensetzungen lässt sich durch gleichzeitige sequentielle oder parallele Verabreichung der entsprechenden Stoffe eine entsprechende Immunantwort in Säugern, insbesondere Menschen, stimuliert. Die Immunantwort ist dabei insbesondere gegen eine durch ein Pathogen verursachte Infektion gerichtet. Das erfindungsgemässe Adjuvans bzw. die pharmazeutische Zusammensetzung wird vorzugsweise parenteral verabreicht. Das Verfahren zur Stimulierung einer Immunantwort wird vorzugsweise so durchgeführt, dass eine schützende Immunantwort induziert wird.

Die erfindungsgemässe Eisenverbindung in kolloidaler Form kann zur Adjuvantierung einer Antigenlösung verwendet werden sowie zur Herstellung eines Adjuvans wie in den Ansprüchen definiert.

Das erfindungsgemässe Adjuvans kann auch zur Herstellung entsprechender pharmazeutischer Zusammensetzungen prophylaktischen oder therapeutischen Behandlung von Erkrankungen, die durch verschiedene Pathogene hervorgerufen werden, verwendet werden.

Die Erfindung soll nun durch die folgenden Beispiele näher erläutert werden.

### Beispiel 1:

### Herstellung der Eisenverbindung in kolloidaler Form:

Unter Rühren und Erhitzen wurden 4,19 g Eisen-III-Citrat (Merck, Darmstadt, Deutschland) in 100 ml bidest. Wasser gelöst. Dieser Lösung wurde bei Raumtemperatur und unter starkem Rühren 32%ige Natronlauge (Merck) bis zu einem pH von 8,0 zugetropft. Dann wurde noch 30 Min. gerührt. Das gebildete Kolloid wurde hochtourig (30 000 x g) zentrifugiert, der Überstand verworfen und der Niederschlag in bidest. Wasser (entsprechend dem Ausgangsvolumen) resuspendiert. Zentrifugation und Resuspension wurden so lange wiederholt, bis der Überstand nach der Zentrifugation farblos war. Das abzentrifugierte und in Wasser, entsprechend dem Ausgangvolumen an Eisencitratlösung, resuspendierte Material wurde durch ein 0.45 µm Sterilfilter gepresst, und die so erhaltene sterile Eisenverbindung in kolloidaler Form bei 4°C bis zur Verwendung gelagert. Das Kolloid war unter diesen Umständen länger als 1 Jahr stabil.

### Beispiel 2:

### Bestimmung der Teilchengrösse des nach Beispiel 1 erhaltenen Kolloids:

In einem Partikelzählgerät (ZetaSizer Typ 4 der Fa. Malvern, Worcestershire, England) wurde die Partikelgrösse in Nanometern bestimmt. In vier unabhängigen Versuchsansätzen wurden Partikelgrössen um 200 nm gemessen (Tabelle 1). Mit Hilfe der Atomabsorptionsmessung wurde der Eisengehalt der Kolloide bestimmt (Tabelle 1). Im Mittel enthielten die Eisenkolloidpräparationen 7,6 Millimol Eisen pro Liter. Die Viskosität des Eisenkolloids wurde durch Messung der Strömungsgeschwindigkeit durch eine Kapillare gemessen. Eine 2 ml Injektionsspritze mit einem Dreiweghahn und einer Kanüle (23 G x 1 1/4 inch) versehen, wurde mit 2 ml Probe befüllt. Nach öffnen des Hahnes wurde die Zeit bestimmt, die für das Auslaufen der 2 ml Probe benötigt wird. Es zeigte sich, dass Eisenkolloid im Vergleich zu Wasser, aber auch im Vergleich zu einer 0,2%igen Aluminiumhydroxidsuspension, wie sie für die parenterale Applikation verwendet wird, eine geringere Durchflussgeschwindigkeit hatte. Wie aus Tabelle 2 ersichtlich ist, entspricht die Durchflussgeschwindigkeit des Eisenkolloids eher der einer 20%igen Glycerinlösung bzw. einer 10%igen Proteinlösung (Bovines Serum-Albumin, BSA) als jener von Wasser, wie dies für Aluminiumhydroxid der Fall ist.

**Tabelle 1**

| **Eigenschaften des Eisenkolloids** | | |
|---|---|---|
| Versuchsnummer | Partikelgrösse (nm) | Fe-Gehalt (mmol/l) |
| # 1 | 166 | 10,3 |
| # 2 | 199 | 5,2 |
| # 3 | 184 | 6,1 |
| # 4 | 215 | 8,7 |
| Mittelwert | 191,0 | 7,6 |
| SD | 20,9 | 2,3 |

**Tabelle 2:**

| **Durchflussdauer von 2 ml Probe durch eine Kanüle** | | |
|---|---|---|
| Probe | Durchflussdauer Absolut | Durchflussdauer bezogen auf Wasser |
| Wasser | 125 sec | -- |
| 0,2 % Al(OH)₃ | 136 sec | 11 sec |
| Eisenkolloid | 246 sec | 121 sec |
| 10 % BSA | 341 sec | 216 sec |
| 20 % Glycerin | 324 sec | 299 sec |

### Beispiel 3:

### Proteinadsorption

Ein Eisenkolloid, erfindungsgemäss wie in Beispiel 1 beschrieben hergestellt, wurde mit verschieden konzentrierten Proteinlösungen gemischt, wozu gereinigtes humanes IgG als Modell-Protein verwendet wurde, 16 Stunden bei 4°C geschüttelt oder unter Drehen inkubiert und dann in einer Heraeus Biofuge B 30 min bei 11 000 rpm und 4°C Temperatur abzentrifugiert. Der Überstand wurde anschliessend durch Radialimmundiffusion nach der Methode von Mancini et al. (Immunochemistry 2 (1965) 235-54) auf nicht gebundenes Protein überprüft. Die Proteinbindungsfähigkeit des Eisenkolloids ergab sich dann durch Subtraktion dieses Wertes von jenem für insgesamt angebotenes Protein

Zum Vergleich wurde auch eine Aluminiumhydroxidlösung, die auf die gleiche molare Konzentration wie das Eisenkolloid eingestellt worden ist, mit Protein gemischt und auf die gleiche Art wie oben beschrieben behandelt.

Tabelle 3 zeigt, dass das erfindungsgemässe Eisenkolloid eine höhere Kapazität hat, Protein zu binden.

**Tabelle 3**

| **Proteinadsorption** | | | | |
|---|---|---|---|---|
| Protein angeboten | Protein an Eisenkollid gebunden | | Protein an Aluminiumhydroxid gebunden | |
| µg | µg | % gebunden | µg | % gebunden |
| 791 | 391 | 49 | 299 | 38 |
| 357 | 256 | 72 | 180 | 50 |
| 166 | 166 | 100 | 113 | 68 |

### Beispiel 4:

### Intradermale Applikation von Eisenkolloid als Adjuvans:

Die adjuvanten Eigenschaften des erfindungsgemäss hergestellten Eisenkolloids wurden nach einer intradermalen Applikation bei balb/c-Mäusen getestet. Jeweils fünf Mäuse pro Gruppe wurden mit 20 ng Tetanus-Toxoid ohne adjuvans bzw. mit 20ng Tetanus-Toxoid, das an Eisenkolloid gebunden war, immunisiert. Für die Bindung wurde 1 Teil Tetanus-Toxoidlösung mit 9 Teilen Eisenkolloid gemischt und unter Schütteln 16 h bei 4°C inkubiert. Mit beiden Präparationen wurden jeweils 2 Immunisierungen im Abstand von 4 Wochen durchgeführt. Zwei Wochen nach der zweiten Immunisierung wurde den Mäusen Blut abgenommen und das Serum auf Tetanus-Toxoid-Antikörper der IgG-Klasse untersucht.

Dazu wurden Nunc-MaxiSorp F96-ELISA-Platten mit 100 µl einer 10 µg/ml Tetanus-Toxoidlösung in Carbonatpuffer (pH 9,6) befüllt. Nach 16 h Inkubation bei 4°C wurde ungebundenes Tetanus-Toxoid abgesaugt und freie Bindungsstellen an der Platte mit 2 % BSA (bovines Serumalbumin) in PBS (phosphatgepufferte Kochsalzlösung) gesättigt. Nach Inkubation mit den Proben bzw. mit einem internen Positiv-Kontrollserum in verschiedenen Verdünnungssstufen (16 h/4°C) wurde mit Peroxidase-markiertem Ziege-anti-Maus-IgG (Accurate Chem., Westbury, NY, USA, 1:50 000 verdünnt) 90 min bei 37°C inkubiert und mit ortho-Phenylendiamin (3 mg/ml) detektiert. Vor dem Messen bei 490 nm in einem Nunc-ImmunoReader wurde die Reaktion mit 2 N Schwefelsäure gestoppt. Zur Auswertung wurde die höchste Probenverdünnung herangezogen, deren optische Dichte nach der Farbreaktion grösser als 0,2 ist. Der Reziprokwert dieser Verdünnung entspricht dem gesuchten Titer der Probe.

Tabelle 4 zeigt, dass Eisenkolloid die Immunantwort selbst auf diese geringe Dosis von Tetanus-Toxoid massiv steigert.

**Tabelle 4:**

| **Intradermale Applikation von Eisenkolloid als Adjuvans** | | |
|---|---|---|
| Antigen | Adjuvans | IgG anti-Tetanus-Toxoid-Titer |
| 20 ng Tetanus-Toxoid | - | 100 |
| 20 ng Tetanus-Toxoid | Eisenkolloid | 64 000 |
| 0 ng (NaCl-Lösung) | - | < 50 |

### Beispiel 5

### Orale Verabreichung von Eisenkolloid als Adjuvans

Eine Verstärkung der Immunantwort durch das erfindungsgemäss hergestellte Eisenkolloid wurde nach oraler Verabreichung von FSME-Antigen (Frühsommer-Meningoenzephalitis-Virus-Antigen, Immuno AG) untersucht. Dazu wurden C57/BL6 Mäuse dreimal in einwöchigem Abstand mit FSME-Antigen allein bzw. mit an Eisenkolloid gebundenem Antigen (für die Herstellung einer Vakzine wurde eine Mischung aus gleichen Teilen FSME-Antigenlösung in PBS und Eisenkolloid 16 h bei 4°C unter Schütteln inkubiert) mittels Intubation immunisiert. Zwei Wochen nach der letzten Immunisierung wurde den Versuchstieren Blut und Speichel abgenommen und auf Antikörper der Klasse IgG und IgA gegen FSME mit Hilfe eines Enzym-Immunassays getestet.

Um auf Antikörper der IgG-Klasse zu prüfen, werden Nunc-MaxiSorp F96-ELISA-Platten mit 100µl einer 5µg/ml FSME-Antigenlösung in Carbonatpuffer, pH 9,6, befüllt. Nach 16 h Inkubation bei 4°C wird ungebundenes FSME-Antigen abgesaugt und freie Bindungsstellen an der Platte mit 2% BSA in PBS gesättigt. Nach Zugabe der Proben bzw. eines Positiv-Kontrollserums in verschiedenen Verdünnungsstufen wird 2h bei 37°C inkubiert. Dann wird Peroxidase-markiertes Ziege-anti-Maus-IgG (Accurate Chem., Westbury, NY, USA, 1:150.000 verdünnt) zugegeben, weitere 90 min bei 37°C inkubiert und danach mit ortho-Phenylendiamin (3 mg/ml) detektiert. Vor dem Messen bei 490 nm im Microtitterplatten-Reader wird die Reaktion mit 2 N Schwefelsäure gestoppt. Zur Auswertung wird die höchste Probenverdünnung herangezogen, deren optische Dichte nach der Farbreaktion grösser als 0,2 ist. Der Reziprokwert dieser Verdünnung ist der gesuchte Titer der Probe.

Zur Prüfung auf Antikörper der Klasse IgA wurden Testsera oder Speichel 1:10 mit PBS + 1% BSA verdünnt, mit einer Protein G Sepharose-Suspension (Pharmacia Biotech, Uppsala, Schweden) versetzt und 1 h unter Schütteln inkubiert, um den Grossteil der in der Probe enthaltenen IgG-Antikörper durch Bindung an Protein G zu entfernen. Eventuelle Kompetitierung des IgG mit der Bindung des IgA an das FSME-Antigen im folgenden ELISA werden dadurch minimiert.

Der Enzym-Immunassay auf IgA-Antikörper gegen FSME wurde in Analogie zum Test auf IgG durchgeführt. Die Detektion des gebundenen IgA erfolgt jedoch durch Reaktion mit einem Biotin-markierten Ziege-anti-Maus-IgA-Reagens (1:50.000 verdünnt, 60 min/37°C, Firma Southern Biotechnology, Birmingham, AL/USA) und nachfolgend mit Peroxidase-markiertem Streptavidin (Firma Zymed, 1:2.000 verdünnt, Inkubation: 60 min/37°C).

Tabelle 5 zeigt, dass Eisenkolloid die humorale Immunreaktion, gemessen durch Antigen-spezifische Antikörpertiter, verstärkt. Wie erwartet, führt Eisenkolloid allein verabreicht zu keiner Bildung spezifischer Antikörper.

**Tabelle 5**

| **Orale Applikation von Eisenkolloid als Adjuvans** | | | | |
|---|---|---|---|---|
| Antigen | Adjuvans | FSME-spezifische Antikörpertiter Serum Speichel | | |
| | | IgG | IgA | IgA |
| 120µg FSME Antigen | - | 12800 | 640 | 80 |
| 120µg FSME Antigen | Eisenkolloid | 25600 | 2560 | 160 |
| PBS | Eisenkolloid | > 100 | 20 | > 20 |

### Vergleichsversuch 1:

Entsprechend dem in EP-363 835 beschriebenen Verfahren wurde eine Eisen-III-chloridlosung (FeCl₃, Merck, Darmstadt, Deutschland) unter Rühren mit Natronlauge bis zu einem pH von 7,0 versetzt. Das auf diese Weise erhaltene Material liegt als Niederschlag vor (Eisenhydroxid-Gel).

Zur Homogenisierung dieses Niederschlags wurde - wie auch in EP-363 835 beschrieben - eine Ultraturrax-Behandlung durchgeführt. Durch eine anschliessende Partikelgrössenmessung im Malvern-ZetaSizer wurde die Partikelgrösse mit 1.000 nm bestimmt. Damit zeigte sich, dass das nach EP-363 835 hergestellte Eisenhydroxid auch nach einer Ultraturrax-Behandlung keine Partikelgrössen kolloidaler Dimensionen aufwies, wie sie z.B. in der Beschreibung der vorliegenden Anmeldung angegeben sind (s. z.B. Anspruch 5).

Durch diesen Vergleichsversuch konnte gezeigt werden, dass das entsprechend EP-363 835 erhaltene Eisenhydroxid als Gel vorliegt und nicht in der erfindungsgemässen kolloidalen Form.

### Vergleichsversuch 2:

Der nachfolgende Versuch zeigt die Proteinadsorption gemäss der Erfindung im Vergleich zur Proteinadsorption an ein gemäss EP-363 835 mit Hilfe von Ultraturrax homogenisiertes Eisenhydroxid. Die bei dem Versuch erhaltenen Daten sind in der nachfolgenden Tabelle A zusammengestellt:

**Tabelle A**

| Proteinadsorption (nach Einstellung auf gleichen Eisengehalt) | | | | |
|---|---|---|---|---|
| Protein angeboten | Protein an erfindungsgemässem Eisen-Kolloid gebunden | | Protein an Eisenhydroxid-Gel gemäss EP-363 835 (nach Ultraturrax) gebunden | |
| µg | in µg bzw. | % gebunden | in µg bzw. | % gebunden |
| 813 | 510 | 63 | 235 | 29 |
| 449 | 360 | 80 | 212 | 48 |
| 199 | 193 | 97 | 185 | 80 |

Die vorstehende Tabelle zeigt deutlich, dass bei vergleichbarem Eisengehalt der beiden Eisenpräparationen und gleicher Menge an angebotenem Protein in hoher Konzentration das erfindungsgemässe Kolloid mehr als das Doppelte der Menge an Protein adsorbieren kann, als dies bei dem gemäss EP-363 835 hergestellten Gel der Fall ist.

## Patentansprüche

1. Verwendung einer Eisenverbindung in kolloidaler Form als Adjuvans, **dadurch gekennzeichnet, daß** sie als Kolloidale lösung vorliegt und daß die Teilchengröße der Kolloide 1nm bis 500 nm beträgt, mit der Maßgabe, daß die Eisenverbindung kein magnetisches Eisenoxid ist.

2. Verwendung nach Anspruch 1, **dadurch gekennzeichnet, dass** das Eisen in der Eisenverbindung in dreiwertiger Form vorliegt.

3. Verwendung nach einem der Ansprüche 1 bis 2, **dadurch gekennzeichnet, dass** die Eisenverbindung im wesentlichen Eisenhydroxid ist.

4. Verwendung nach einem der Ansprüche 1 bis 3, **dadurch gekennzeichnet, dass** sie als wässerige Lösung vorliegt.

5. Verwendung nach einem der Ansprüche 1 bis 3, in einer Wasser-in-Öl Emulsion.

6. Verwendung nach einem der Ansprüche 1 bis 5, **dadurch gekennzeichnet, dass** die Teilchengrösse der Kolloide 100 nm bis 500 nm, und bevorzugt ca. 200 nm, beträgt.

7. Verwendung nach einem der Ansprüche 1 bis 6, **dadurch gekennzeichnet, dass** sie in einer für die parenterale Verabreichung geeigneten Form vorliegt.

8. Verwendung nach einem der Ansprüche 1 bis 6, **dadurch gekennzeichnet, dass** sie in einer für die mukosale Verabreichung, vorzugsweise für die orale Verabreichung, geeigneten Form vorliegt.

9. Adjuvans, hergestellt unter Verwendung einer Eisenverbidung gemäß einem der Ansprüche 1 bis 8 **dadurch gekennzeichnet, dass** es zusätzlich wenigstens ein Interleukin, Interferon, ein anderes Cytokin oder Mischungen davon enthält.

10. Adjuvans nach Anspruch 9, **dadurch gekennzeichnet, dass** das Cytokin an das Kolloid adsorbiert vorliegt.

11. Pharmazeutische Zusammensetzung, **dadurch gekennzeichnet, dass** sie wenigstens ein Procein, Peptid, Polysaccharid, und/oder eine Nukleinsäure, abgeleitet von einem Virus, Bakterium und/oder Parasiten, und ein Adjuvans, enthaltend eine Eisenverbindung in kolloidaler lösung, wobei die Teilchengröße der kolloide 1nm bis 500nm betrügt, gegebenenfalls zusammen mit einem pharmazeutischen Träger und/oder Verdünner, enthält.

12. Pharmazeutische Zusammensetzung nach Anspruch 11, **dadurch gekennzeichnet, dass** das Virus ausgewählt ist aus der Gruppe bestehend aus Retroviren, Hepatitisviren, Flaviviren, Herpesviren, Influenzaviren, Poxviren, Morbilliviren, Enteroviren (Poliovirus) oder Paramyxoviren.

13. Pharmazeutische Zusammensetzung nach Anspruch 11 oder 12, **dadurch gekennzeichnet, dass** das Virus als Ganzvirus, virus-ähnliches Partikel oder Virus-Spaltprodukt vorliegt.

14. Pharmazeutische Zusammensetzung nach einem der Ansprüche 11 bis 13, **dadurch gekennzeichnet, dass** das Ganzvirus inaktiviert, attenuiert oder ein rekombinantes Virus ist.

15. Pharmazeutische Zusammensetzung nach einem der Ansprüche 11 bis 14, **dadurch gekennzeichnet, dass** das Protein und/oder Peptid und/oder Polysaccharid und/oder die Nukleinsäure an ein Adjuvans hergestellt unter Verweudung einer Eisenverbindung gemäss einem der Ansprüche 1 bis 8, oder an ein Adjurens gemäss einem der Ansprüche 9 oder 10 adsorbiert vorliegt.

16. Pharmazeutische Zusammensetzung nach Anspruch 11, **dadurch gekennzeichnet, dass** das Bakterium ausgewählt ist aus der Gruppe bestehend aus E.coli, Bordetella, Borrelia, Pseudomonas, Haemophilus, Mycobakterien, Streptokokken, Salmonellen, Helicobacter oder Clostridien.

17. Pharmazeutische Zusammensetzung nach Anspruch 11, **dadurch gekennzeichnet, dass** der Parasit ausgewählt ist aus der Gruppe bestehend aus Amaebida, Trypanosoma und Plasmodium.

18. Pharmazeutische Zusammensetzung nach einem der Ansprüche 11 bis 17, **dadurch gekennzeichnet, dass** sie eine Impfstoffzusammensetzung ist.

19. Pharmazeutische Zusammensetzung nach Anspruch 18, **dadurch gekennzeichnet, dass** die Impfstoffzusammensetzung ein Antigen eines Virus, Bakteriums und/oder Parasiten enthält.

20. Pharmazeutische Zusammensetzung nach einem der Ansprüche 11 bis 19, **dadurch gekennzeichnet, dass** sie für die parenterale Verabreichung geeignet ist.

21. Pharmazeutische Zusammensetzung nach einem der Ansprüche 11 bis 19, **dadurch gekennzeichnet, dass** sie für die mukosale Verabreichung, vorzugsweise für die orale Verabreichung, geeignet ist.

22. Pharmazeutische Zusammensetzung nach einem der Ansprüche 11 bis 21, **dadurch gekennzeichnet, dass** sie zusätzlich ein Interleukin, Interferon, ein anderes Cytokin oder Mischungen davon enthält.

23. Verwendung eines Adjuvans, hergestellt unter Verwendung einer Einsenverbindung gemäß einem der Ansprüche 1 bis 8 zur Herstellung einer pharmazeutischen Zusammensetzung zur prophylaktischen oder therapeutischen Behandlung von Erkrankungen, die durch verschiedene Pathogene hervorgerufen werden.

## Claims

1. The use of an iron compound in colloidal form as an adjuvant, **characterized in that** it is provided as a colloidal solution and that the particle size of the colloids ranges from 1 nm to 500 nm, with the proviso that the iron compound is not a magnetic iron oxide.

2. The use according to claim 1, **characterized in that** the iron is provided in the iron compound in trivalent form.

3. The use according to any of claims 1 to 2, **characterized in that** the iron compound essentially is an iron hydroxide.

4. The use according to any of claims 1 to 3, **characterized in that** it is provided as an aqueous solution.

5. The use according to any of claims 1 to 3 in a water-in-oil emulsion.

6. The use according to any of claims 1 to 5, **characterized in that** the particle size of the colloids ranges from 100 nm to 500 nm and preferably is about 200 nm.

7. The use according to any of claims 1 to 6, **characterized in that** it is provided in a form suitable for parenteral administration.

8. The use according to any of claims 1 to 6, **characterized in that** it is provided in a form suitable for mucosal administration, preferably for oral administration.

9. An adjuvant, prepared by using an iron compound according to any of claims 1 to 8, **characterized in that**, in addition, it comprises at least an interleukin, an interferon, another cytokin or mixtures thereof.

10. An adjuvant according to claim 9, **characterized in that** the cytokin is provided as adsorbed onto the colloid.

11. A pharmaceutical composition, **characterized in that** it comprises at least a protein, a peptide, a polysaccharide and/or a nucleic acid, derived from a virus, a bacterium and/or a parasite, and an adjuvant comprising an iron compound in a colloidal solution, with the particle size of the colloids ranging from 1 nm to 500 nm, optionally together with a pharmaceutical vehicle and/or a diluting agent.

12. A pharmaceutical composition according to claim 11, **characterized in that** the virus is selected from the group consisting of retroviruses, hepatitis viruses, flaviviruses, herpes viruses, influenza viruses, pox viruses, morbilliviruses, enteroviruses (polio viruses) or paramyxoviruses.

13. A pharmaceutical composition according to claim 1 or 12, **characterized in that** the virus is provided as a whole virus, a virus-like particle or a virus-cleaving product.

14. A pharmaceutical composition according to any of claims 11 to 13, **characterized in that** the whole virus is inactivated, attenuated or is a recombinant virus.

15. A pharmaceutical composition according to any of claims 11 to 14, **characterized in that** the protein and/or the peptide and/or the polysaccharide and/or the nucleic acid are provided as adsorbed onto an adjuvant, prepared by using an iron compound according to any of claims 1 to 8, or onto an adjuvant according to any of claims 9 or 10.

16. A pharmaceutical composition according to claim 11, **characterized in that** the bacterium is selected from the group consisting of E. coli, Bordetella, Borrelia, Pseudomonas, Haemophilus, Mycobacteria, Streptococci, Salmonella, Helicobacter or Clostridii.

17. A pharmaceutical composition according to claim 11, **characterized in that** the parasite is selected from the group consisting of Amaebida, Trypanosoma and Plasmodium.

18. A pharmaceutical composition according to any of claims 11 to 17, **characterized in that** it is a vaccine composition.

19. A pharmaceutical composition according to claim 18, **characterized in that** the vaccine composition comprises an antigen of a virus, a bacterium and/or a parasite.

20. A pharmaceutical composition according to any of claims 11 to 19, **characterized in that** it is suitable for parenteral administration.

21. A pharmaceutical composition according to any of claims 11 to 19, **characterized in that** it is suitable for mucosal administration, preferably for oral administration.

22. A pharmaceutical composition according to any of claims 11 to 21, **characterized in that**, in addition, it comprises an interleukin, an interferon, another cytokin or mixtures thereof.

23. The use of an adjuvant, prepared by using an iron compound according to any of claims 1 to 8, for producing a pharmaceutical composition for the prophylactic or therapeutic treatment of diseases caused by different pathogens.

## Revendications

1. Utilisation d'un composé du fer sous forme colloïdale comme adjuvant **caractérisée en ce qu'**il est présent sous forme de solution colloïdale et **en ce que** la taille de particules des colloïdes est 1 nm à 500 nm, à condition que le composé du fer ne soit pas un oxyde de fer magnétique.

2. Utilisation selon la revendication 1 **caractérisée en ce que** le fer dans le composé du fer est présent sous forme trivalente.

3. Utilisation selon l'une des revendications 1 à 2 **caractérisée en ce que** le composé du fer est essentiellement de l'hydroxyde de fer.

4. Utilisation selon l'une des revendications 1 à 3 **caractérisée en ce qu'**elle est sous forme de solution aqueuse.

5. Utilisation selon l'une des revendications 1 à 3 dans une émulsion eau dans huile.

6. Utilisation selon l'une des revendications 1 à 5 **caractérisée en ce que** la taille de particules des colloïdes est 100 nm à 500 nm et de préférence d'environ 200 nm.

7. Utilisation selon l'une des revendications 1 à 6 **caractérisée en ce qu'**elle est sous une forme appropriée pour l'administration parentérale.

8. Utilisation selon l'une des revendications 1 à 6 **caractérisée en ce qu'**elle est sous une forme appropriée pour l'administration muqueuse, de préférence pour l'administration orale.

9. Adjuvant produit au moyen d'un composé du fer selon l'une des revendications 1 à 8 **caractérisé en ce qu'**il contient en outre au moins une interleukine, un interféron, une autre cytokine ou des mélanges de ceux-ci.

10. Adjuvant selon la revendication 9 **caractérisé en ce que** la cytokine est adsorbée sur le colloïde.

11. Composition pharmaceutique **caractérisée en ce qu'**elle contient au moins une protéine, un peptide, un polysaccharide et/ou un acide nucléique issus d'un virus, d'une bactérie et/ou de parasites, et un adjuvant contenant un composé du fer en solution colloïdale, où la taille de particule des colloïdes est 1 nm à 5 nm, éventuellement en même temps qu'un support et/ou diluant pharmaceutique.

12. Composition pharmaceutique selon la revendication 11 **caractérisée en ce que** le virus est choisi dans le groupe consistant en les rétrovirus, les virus de l'hépatite, les flavivirus, les herpèsvirus, les virus de la grippe, les poxvirus, les morbillivirus, les entérovirus (poliovirus) et les paramyxovirus.

13. Composition pharmaceutique selon la revendication 11 ou 12 **caractérisée en ce que** le virus est présent sous forme de virus complet de particule analogue à un virus ou de produit de clivage d'un virus.

14. Composition pharmaceutique selon l'une des revendications 11 à 13 **caractérisée en ce que** le virus complet est inactivé, atténué ou est un virus recombiné.

15. Composition pharmaceutique selon l'une des revendications 11 à 14 **caractérisée en ce que** la protéine et/ou le peptide et/ou le polysaccharide et/ou l'acide nucléique sont adsorbés à un adjuvant produit au moyen d'un composé du fer selon l'une des revendications 1 à 8, ou à un adjuvant selon l'une des revendications 9 et 10.

16. Composition pharmaceutique selon la revendication 11 **caractérisée en ce que** la bactérie est choisie dans le groupe consistant en E. coli, Bordetella, Borrelia, Pseudomonas, Haemophilus, les mycobactéries, les streptocoques, les salmonelles, Helicobacter et les clostridies.

17. Composition pharmaceutique selon la revendication 11 **caractérisée en ce que** le parasite est choisi dans le groupe consistant en Amoeba, Trypanosoma et Plasmodium.

18. Composition pharmaceutique selon l'une des revendications 11 à 17 **caractérisée en ce qu'**il s'agit d'une composition vaccinale.

19. Composition pharmaceutique selon la revendication 18 **caractérisée en ce que** la composition vaccinale contient un antigène d'un virus, d'une bactérie et/ou de parasites.

20. Composition pharmaceutique selon l'une des revendications 11 à 19 **caractérisée en ce qu'**elle convient pour l'administration parentérale.

21. Composition pharmaceutique selon l'une des revendications 11 à 19 **caractérisée en ce qu'**elle convient pour l'administration muqueuse, de préférence pour l'administration orale.

22. Composition pharmaceutique selon l'une des revendications 11 à 21 **caractérisée en ce qu'**elle contient en outre une interleukine, un interféron, une autre cytokine ou des mélanges de ceux-ci.

23. Utilisation d'un adjuvant produit au moyen d'un composé du fer selon l'une des revendications 1 à 8 pour la production d'une composition pharmaceutique pour le traitement prophylactique ou thérapeutique de maladies qui sont provoquées par différents agents pathogènes.
